# EUROPEAN PATENT SPECIFICATION

(11) **EP 1 963 309 B1**
(45) Date of publication and mention of the grant of the patent: **10.11.2010**
(21) Application number: 05825006.9
(22) Date of filing: 01.12.2005
(51) Int. Cl.: C07D 403/10

(54) **METHOD FOR PRODUCING METAL SALTS OF LOSARTAN**
VERFAHREN ZUR HERSTELLUNG VON METALLSALZEN VON LOSARTAN
PROCÉDÉ DE PRODUCTION DE SELS MÉTALLIQUES DE LOSARTAN

(43) Date of publication of application: 03.09.2008
(73) Proprietor: Ulkar Kimya Sanayii Ve Ticaret A.S., Besiktas 34353 Istanbul (TR)
(72) Inventor: ASLAN, Tuncer, 34353 Besiktas Istanbul (TR); GÜLKOK, Yildiz, 34353 Besiktas Istanbul (TR); BEYDEMIR, Meltem, 34353 Besiktas Istanbul (TR); BICER, Tuba, 34353 Besiktas Istanbul (TR); TURHAN, Selda, 34353 Besiktas Istanbul (TR); KÖROGLU, Melek, 34353 Besiktas Istanbul (TR)
(74) Representative: Özbay, Cenk
(86) International application number: PCT/EP2005/012793
(87) International publication number: WO 2007/062675

(56) References cited:
- WO-A-03/093262
- WO-A-2004/066997

## Description

The present invention relates to a method for producing metal salts of Losartan of the following formula 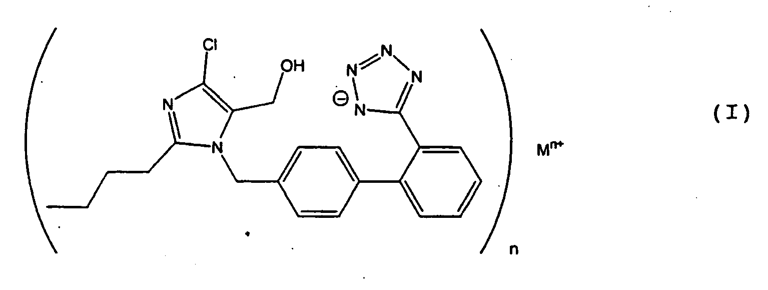 wherein M is any metal and n is an integer, comprising treating Losartan free acid with a basic metal salt in a solvent.

Losartan is a member of a group of pharmacologically active compounds which are generally referred to as "sartans". These compounds are anti-hypertensive agents used to reduce blood pressure. They work by blocking the action of Angiotensin II on its receptor. Angiotensin II mediates among others smooth muscle contraction especially in blood vessels. Angiotensin 11 receptor antagonists block the Angiotensin II receptor and thereby counteract muscle contraction especially in blood vessels. Angiotensin II receptor antagonists therefore act as powerful vasodilators.

Losartan is generally administered in the form of a metal salt. In order to produce such metal salts, Losartan free acid is treated with a basic metal salt in a solvent.

It is for example known from WO 03/093262 to treat Losartan free acid with potassium hydroxide in a solvent consisting essentially of an alcohol selected from the group consisting of isopropyl alcohol, butyl alcohol and isobutyl alcohol and to precipitate the formed Losartan potassium from the solvent.

According to this document the conversion of Losartan free acid to Losartan potassium and the subsequent precipitation can be achieved in a 85 % yield. While 85 % can be considered an acceptable yield, there is always an interest in improving the yield, especially when dealing with high value compounds such as those used in pharmaceuticals.

Furthermore in the method according to WO 03/093262 Losartan free acid is dissolved in an excess of solvent of which part has to be removed in order to precipitate the desired Losartan potassium. Such an extra step of removing excess solvent is time and energy consuming as well as wasteful in resources.

WO 2004/066997 discloses a method for producing a potassium salt of Losartan by treating Losartan free acid with potassium hydroxide in the presence of isopropanol, water and n-heptane.

It is therefore an object of this invention to describe a new method for producing metal salts of Losartan which results in a high yield, is quick to perform as well as more economical in terms of resource and energy use.

It was now surprisingly found that if the solvent comprises a mixture of isopropyl alcohol and *t*-butyl methyl ether significant improvements in the yield can be achieved. Furthermore the reaction can be performed at a higher initial concentration of Losartan free acid compared to the state of the art. The metal salt of Losartan will therefore precipitate from the solvent without the need to remove some of the solvent.

The object is therefore achieved by a method for producing metal salts of Losartan by treating Losartan free acid with a basic metal salt in a solvent wherein the solvent comprises a mixture of isopropyl alcohol and *t*-butyl methyl ether.

With this method yields of 90 % and more can be achieved and the desired metal salt precipitates directly from the initial solution therefore making the step of removing parts of the solvent superfluous. Therefore the new process does not only increase the yield but is also quicker as well as more economical with regards to energy and resources used.

The expression "basic metal salt" as it is used herein comprises all compounds, which are able to deprotonate Losartan and comprise at least one metal ion. Non-limiting examples for such compounds include metal hydroxides and metal alkoxides.

In an embodiment of the invention the basic metal salt is selected from the basic alkali and basic earth alkali metal salts.

The use of basic alkali and basic earth alkali metal salts is preferred since they are usually cheaply available and they result in metal salts of Losartan, which have been shown to be generally well tolerated.

In an embodiment of the invention the basic metal salt is selected from the metal hydroxides and in particular is potassium hydroxide.

The use of metal hydroxides has the advantage that the only byproduct of their reaction with Losartan free acid is water. Therefore no extra step of purifying the resulting product is necessary. The use of potassium hydroxide is particularly preferred, since this forms Losartan potassium which has been shown to be the preferred metal salt of Losartan.

In an embodiment of the invention the solvent comprises at least 25 % by volume of *t*-butyl methyl ether, preferably it comprises 25 to 75 % by volume, particularly preferable 35 to 65 % by volume and especially 45 to 55 % by volume of *t*-butyl methyl ether.

When reacting Losartan free acid with a basic metal salt a balance must be struck between the solubility of Losartan free acid which is more soluble in the less polar *t-*butyl methyl ether and the solubility of the basic metal salt which is more soluble in the more polar isopropyl alcohol. The above-mentioned mixture ranges form a good balance between those two conflicting solubilities therefore resulting in particularly high yields.

In a further embodiment of the invention the solvent comprises water, particularly 0.5 to 2.5 % by volume and especially 0.75 to 1.5 % by volume of water.

It has been shown that the addition of small quantities of water further improves the yield. It is believed that this is due to the fact that small quantities of water are effective in improving the solubility of the basic metal salt while at the same time not significantly affecting the solubility of Losartan.

In an embodiment of the invention Losartan free acid is treated with the basic metal salt at a temperature from 35 to 75°C, preferably at a temperature from 45 to 65°C and especially at a temperature from 50 to 60°C.

When reacting large and potentially unstable organic compounds such as Losartan free acid with basic metal salts a balance must be found between the fact that at high temperatures those compounds have the tendency to degrade, resulting in a lower yield and the necessity that the temperature is high enough that the reaction proceeds within a reasonable period of time.

It has been found in the above-mentioned temperature ranges high yields can be achieved in about 5 hours.

In an embodiment of the invention 1.0 to 1.1 molar equivalents of the basic metal salt based on Losartan free acid are used.

When deprotonating larger organic molecules such as Losartan free acid with a basic metal salt a balance must be struck between using enough of the basic metal salt in order to fully deprotonate the large organic molecule and the fact that at high concentrations of the basic metal salt is degraded by an excess of the basic metal salt.

Furthermore if excesses of the basic metal salt are used they can precipitate together with the metal salt of Losartan from the solution thereby leading to an impure product. Any additional purification steps necessitated by this render the process less economical and can lead to significant losses in yield.

At the above named ratio particularly pure products with good to excellent yields can be recovered.

It is understood that the above features and the features described below can be used not only in their described combination but also in other combinations or in isolation without departing from the scope of the invention.

The invention is now further illustrated by means of examples. These examples are not intended to limit the scope of the invention in any way.

### EXAMPLES

### General procedure for the production of Losartan salts

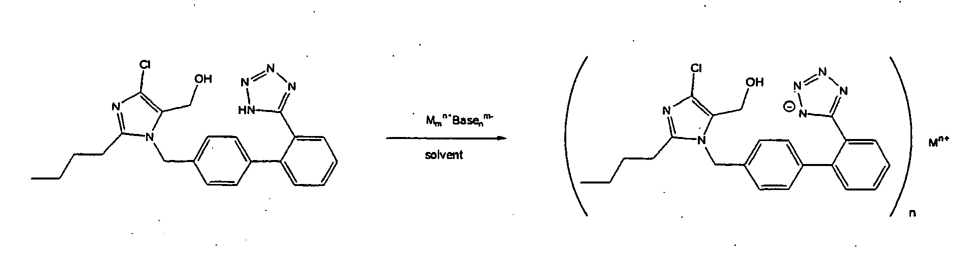

Losartan free acid is added to a mixture comprising isopropyl alcohol, *t*-butyl methyl ether and optionally a small quantity of water. The resulting suspension is optionally heated to 35 to 75°C. In a second flask a basic metal salt is added to isopropyl alcohol and the mixture is heated in order to dissolve the basic metal salt. The solution of the basic metal salt is filtered and added to the suspension of Losartan while stirring. After the addition of the solution of the basic metal salt the solvent preferably comprises at least 25 % *t*-butyl methyl ether. The reaction is stirred for 1 hour at up to 75°C and then if necessary cooled to room temperature. The stirring is continued for 1 to 3 hours at room temperature as the desired metal salt of Losartan precipitates. The resulting solid is collected by filtration and dried to yield the desired metal salt of Losartan.

### EXAMPLE 1

### Preparation of Losartan potassium from Losartan

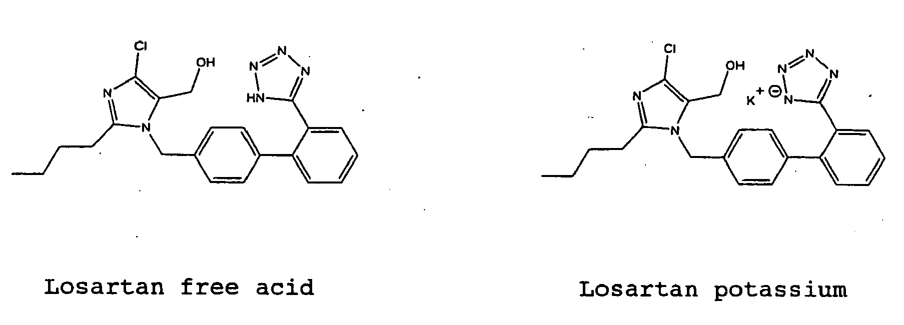

A 850 ml flask equipped with a magnetic stirrer bar, a thermometer and a reflux condenser was charged with 20 ml of isopropyl alcohol, 80 ml of *t*-butyl methyl ether and 20 g (47.3 mmols) of Losartan free acid at room temperature to give a suspension. In a second flask 3.24 g (49.6 mmols) of KOH were dissolved in 60 ml isopropyl alcohol by heating. The KOH solution was filtered and added to above suspension while stirring. At this point the solvent is approximately a 50:50 mixture of isopropyl alcohol and *t*-butyl methyl ether. After the addition was complete the reaction mixture was stirred for 3 hours at room temperature. White crystals started to precipitate which were collected by filtration and dried to yield 19 g (87 % yield) of Losartan potassium with a relative purity of 99 % (HPLC).

### EXAMPLE 2

### Preparation of Losartan potassium from Losartan free acid

A 250 ml flask equipped with a magnetic stirrer bar, a thermometer and a reflux condenser was charged with 20 ml of isopropyl alcohol, 80 ml of *t*-butyl methyl ether, 1 ml of water and 20 g (47.3 mmols) of Losartan free acid at room temperature to give a suspension. In a second flask 3.24 g (49.6 mmols) of KOH were dissolved in 60 ml of isopropyl alcohol by heating. The KOH solution was filtered and added to the above suspension while stirring. After the addition was complete the reaction mixture was stirred for 3 hours at room temperature. White crystals started to precipitate which were collected by filtration and dried to yield 20 g (92 % yield) of Losartan potassium with a relative purity of 99.9 % (HPLC). Compared to EXAMPLE 1, the addition of small amounts of water increases the yield.

### EXAMPLE 3

### Preparation of Losartan potassium from Losartan free acid

A 850 ml flask equipped with a magnetic stirrer bar, a thermometer and a reflux condenser was charged with 10 ml of isopropyl alcohol, 40 ml of *t*-butyl methyl ether, 0.5 ml of water and 10 g (23.6 mmols) of Losartan free acid and the resulting suspension was heated to 55°C. In a second flask 1.62 g (24.8 mmols) of KOH were dissolved in 30 ml of isopropyl alcohol by heating. The KOH solution was filtered and added to the above suspension while stirring. After the addition was complete, the reaction mixture was stirred for 1 hour at 55°C. The reaction mixture was cooled to room temperature over about 1 hour and stirred over night at this temperature. The precipitated white crystals were collected by filtration and dried to yield 10.14 g (93 % yield) of Losartan potassium with a relative purity of 99 % (HPLC).

### EXAMPLE 4

### Preparation of Losartan potassium from Losartan free acid

A 850 ml flask equipped with a magnetic stirrer bar, a thermometer and a reflux condenser was charged with 10 ml of isopropyl alcohol, 13.3 ml of *t*-butyl methyl ether, 0.5 ml of water and 10 g (23.6 mmols) of Losartan free acid at room temperature and the resulting suspension was heated to 55°C. In a second flask 1.62 g (24.8 mmols) of KOH were dissolved in 30 ml of isopropyl alcohol by heating. The KOH solution was filtered and then added to the above suspension while stirring. At this point the solvent is approximately a 75:25 mixture of isopropyl alcohol and *t*-butyl methyl ether. After the addition was complete the reaction mixture was stirred for 1 hour at 55°C. The reaction mixture was cooled to room temperature over about 1 hour and stirred over night at this temperature. The precipitated white crystals were collected by filtration and dried to yield 9.9 g (91 % yield) of Losartan potassium with a relative purity of 99.9 % (HPLC).

## Claims

1. Method for producing metal salts of Losartan of the following formula 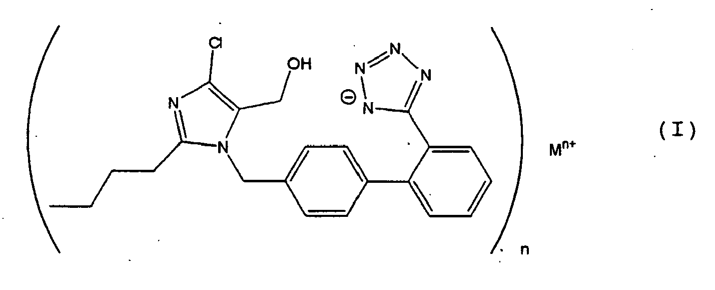 wherein M is any metal and n is an integer;
comprising treating Losartan free acid with a basic metal salt in a solvent,
**characterized in that** the solvent comprises a mixture of isopropyl alcohol and *t*-butyl methyl ether.

2. Method according to claim 1, **characterized** that the basic metal salt is selected from the basic alkali and basic earth alkali metal salts.

3. Method according to claim 1 or 2, **characterized** that the basic metal salt is selected from the metal hydroxides.

4. Method according to claim 3, **characterized in that** the basic metal salt is potassium hydroxide.

5. Method according to any one of claims 1 to 4, **characterized in that** the solvent comprises at least 25% by volume of *t*-butyl methyl ether.

6. Method according to claim 5, **characterised in that** the solvent comprises 25 to 75 % by volume of *t*-butyl methyl ether.

7. Method according to claim 6, **characterized in that** the solvent comprises 35 to 65 % by volume of *t*-butyl methyl ether.

8. Method according to claim 7, **characterized in that** the solvent comprises 45 to 55 % by volume of *t*-butyl methyl ether.

9. Method according to any one of claims 1 to 8, **characterized in that** the solvent further comprises water.

10. Method according to claim 9, **characterized in that** the solvent comprises 0.5 to 2.5 % by volume of water.

11. Method according to claim 10, **characterized in that** the solvent comprises 0.75 to 1.5 % by volume of water.

12. Method according to any one of claims 1 to 11, **characterized in that** Losartan free acid is treated with the basic metal salt at a temperature from 35 to 75°C.

13. Method according to claim 12, **characterized in that** Losartan free acid is treated with the basic metal salt at a temperature from 45 to 65°C.

14. Method according to claim 13, **characterized in that** Losartan free acid is treated with the basic metal salt at a temperature from 50 to 60°C.

15. Method according to any one of claims 1 to 14, **characterized in that** 1.0 to 1.1 molar equivalents of the basic metal salt based on Losartan free acid are used.

## Patentansprüche

1. Verfahren zur Herstellung von Metallsalzen des Losartans mit der folgenden Formel: 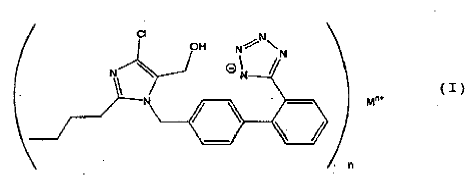 wobei M das jeweilige Metal und n eine Ganzzahl sind;
und das Verfahren die Behandlung der freien Säure des Losartans mit einem basischen Metallsalz in einem Lösungsmittel umfasst,
**dadurch gekennzeichnet, dass** das Lösungsmittel ein Gemisch aus Isopropylalkohol und t-Butylmethylether enthält.

2. Verfahren nach Anspruch 1, **dadurch gekennzeichnet, dass** das basische Metallsalz aus basischen Alkali- oder basischen Erdalkalimetallsalzen ausgewählt wird.

3. Verfahren nach Anspruch 1 oder 2, **dadurch gekennzeichnet, dass** das basische Metallsalz aus Metallhydroxiden ausgewählt wird.

4. Verfahren nach Anspruch 3, **dadurch gekennzeichnet, dass** basische Metallsalz das Potassiumhydroxid ist.

5. Verfahren nach einem der Ansprüche 1 bis 4, **dadurch gekennzeichnet, dass** das Lösungsmittel volumenmäßig mindestens 25% t-Butylmethylether enthält.

6. Verfahren nach Anspruch 5, **dadurch gekennzeichnet, dass** das Lösungsmittel volumenmäßig 25 bis 75 % t-Butylmethylether enthält.

7. Verfahren nach Anspruch 6, **dadurch gekennzeichnet, dass** das Lösungsmittel volumenmäßig 35 bis 65% t-Butylmethylether enthält.

8. Verfahren nach Anspruch 7, **dadurch gekennzeichnet, dass** das Lösungsmittel volumenmäßig 45 bis 55% t-Butylmethylether enthält.

9. Verfahren nach einem der Ansprüche 1 bis 8, **dadurch gekennzeichnet, dass** das Lösungsmittel ferner Wasser enthält.

10. Verfahren nach Anspruch 9, **dadurch gekennzeichnet, dass** das Lösungsmittel volumenmäßig 0.5 bis 2.5% Wasser enthält.

11. Verfahren nach Anspruch 10, **dadurch gekennzeichnet, dass** das Lösungsmittel volumenmäßig 0.75 bis 1.5% Wasser enthält.

12. Verfahren nach einem der Ansprüche 1 bis 11, **dadurch gekennzeichnet, dass** die freie Säure des Losartans bei einer Temperatur von 35 bis 75°C mit einem basischen Metallsalz behandelt wird.

13. Verfahren nach Anspruch 12, **dadurch gekennzeichnet, dass** die freie Säure des Losartans bei einer Temperatur von 45 bis 65°C mit einem basischen Metallsalz behandelt wird.

14. Verfahren nach Anspruch 13, **dadurch gekennzeichnet, dass** die freie Säure des Losartans bei einer Temperatur von 50 bis 60°C mit einem basischen Metallsalz behandelt wird.

15. Verfahren nach einem der Ansprüche 1 bis 14, **dadurch gekennzeichnet, dass** 1.0 bis 1.1 Moleq. an basischem Metallsalz basierend auf der freien Säure des Losartans eingesetzt wird.

## Revendications

1. Le formule ci-dessous est une procédé de production de sels métalliques de Losartan, où ; 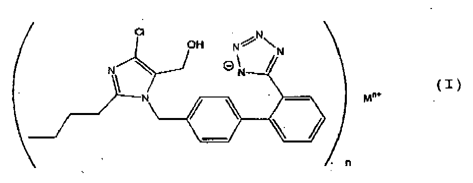 M est un métal quelconque dans lequel n est un nombre entier;
En traitant un acide libre de losartan avec un sel métallique basique dans un solvant, qui comporte l'utilisation d'un solvant qui comprend un mélange d'alcool isoprophylique et d'éther de t-butyle et de méthyle.

2. Procédé selon la revendication 1 **caractérisée par** la sélection du sel métallique basique parmi un sel de métal alcalin ou alcalino-terreux.

3. Procédé selon la revendication 1 ou 2, **caractérisé par** la sélection du sel métallique basique parmi les hydroxydes de métaux alcalins.

4. Procédé selon la revendication 3, **caractérisé par le fait que** le sel métallique basique est de l'hydroxyde de potassium.

5. Procédé suivant n'importe des revendications de 1 à 4, **caractérisé** avec un solvant de teneur en éther de t-butyle et de méthyle au moins de 25 % en volume.

6. Procédé selon la revendication 5, **caractérisé en ce que** la teneur d'éther de t-butyle et de méthyle du solvant soit de l'ordre de 25 a 75 % en volume.

7. Procédé selon la revendication 6, **caractérisé en ce que** la teneur d'éther de t-butyle et de méthyle du solvant soit de l'ordre de 35 a 65 % en volume.

8. Procédé selon la revendication 7, **caractérisé en ce que** la teneur d'éther de t-butyle et de méthyle du solvant soit de l'ordre de 45 à 55 % en volume.

9. Procédé selon l'une quelconque des revendications d'1 à 8, **caractérisé par** l'eau inclus dans le solvant.

10. Procédé suivant la revendication 9, comprenant un solvant de teneur en eau de 0,5 à 2,5 % d'eau en volume.

11. Procédé suivant la revendication 10, constitué par un solvant de teneur en eau de 0,75 à 1,5
% en volume.

12. Procédé selon l'une quelconque des revendications d'1 à 11, **caractérisé par** l'application d'un traitement de l'acide libre de losartan avec le sel métallique basique à une température entre 45 à 65 degrés.

13. Procédé selon la revendication 12, **caractérisé par** l'application d'un traitement de l'acide libre de losartan avec le sel métallique basique à une température entre 45 à 65 degrés.

14. Procédé selon la revendication 13, **caractérisé par** l'application d'un traitement de l'acide libre de losartan avec le sel métallique basique à une température entre 50 à 60 degrés.

15. Procédé selon l'une quelconque des revendications d'1 à 14, **caractérisé par** l'usage d'un sel métallique basique à base d'acide libre de losartan par 1.0 à 1.1 équivalent molaire.
